# EUROPEAN PATENT APPLICATION

(11) **EP 3 412 659 A1**
(43) Date of publication of application: **12.12.2018**
(21) Application number: 17175263.7
(22) Date of filing: 09.06.2017
(51) Int. Cl.: C07D 231/44

(54) **PROCESSES FOR THE MANUFACTURE OF SULFUR-SUBSTITUTED PYRAZOLE DERIVATIVES**

(71) Applicant: SOLVAY SA, 1120 Brussels (BE)
(72) Inventor: The designation of the inventor has not yet been filed
(74) Representative: Mross, Stefan P.M.

(57) **Abstract**

The present invention concerns processes for the manufacture of sulphur-substituted pyrazole derivatives and precursors thereof and processes for the manufacture of agriculturally, insecticidal or pharmaceutically active compound.

## Description

The present invention concerns processes for the manufacture of sulphur-substituted pyrazole derivatives and precursors thereof and processes for the manufacture of agriculturally, insecticidal or pharmaceutically active compound.

Sulphur-substituted pyrazoles, for example C4-sulfur-substituted pyrazoles, can act as useful intermediates in the manufacture of agriculturally, insecticidal or pharmaceutically active ingredients. A C4-trifluorosulfinyl-substituted pyrazole ring is a landmark structure fragment, for example, in 1-arylpyrazoles as disclosed in DE3529829, which are useful as insecticides and arachnicides. Often, the C4-trifluorosulfinyl-substituted pyrazole residue is provided through a disulfide dimer intermediate, such as disclosed in US6881848, which can be technically demanding and may require a multistep synthesis from challenging starting materials such as CF₃Br, which is a banned halon. Patent IN2012CH03583 provides a process for the manufacture of such compounds, which comprises reacting a precursor with RS(O)X in the presence of an SO₂ atmosphere, which is a toxic gas and difficult to handle on a large scale. There is thus a need for an improved synthesis for such C4-sulfur-substituted pyrazoles.

The invention concerns a process for the manufacture of a compound of formula (I) 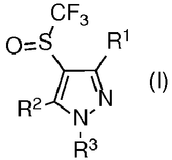 wherein R¹, R² and R³ are defined as in the subsequent description and claims, which comprises at least one of the steps (a) to (d), wherein a compound of formula (II) in a variety of steps into a compound of formula (I).

The invention concerns also a process for the manufacture of a compound of formula (VIII) wherein R¹ is CN and R² is NH₂, wherein a compound of formula (IX), wherein R³ is defined as in the subsequent description and claims, is reacted with 3-amino-3-oxopropanoyl cyanide

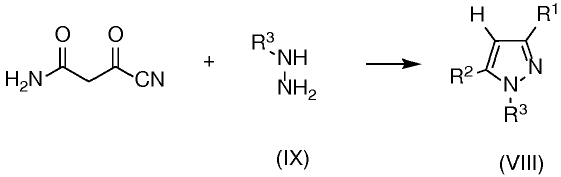

Another object of the present invention is a process for the manufacturing of an agriculturally, insecticidal or pharmaceutically active compound or a composition comprising an agriculturally or pharmaceutically active compound, which comprises at least one of the processes according to the present invention for the manufacture of a compound (I) or (VIII).

In the present invention, designations in singular are in intended to include the plural; for example, "a solvent" is intended to denote also "more than one solvent" or "a plurality of solvents".

All "aspects" of the present invention are combinable.

In the context of the present invention, the term "comprising" is intended to include the meaning of "consisting of'.
When a double bond is depicted in a particular E/Z geometry, this is intended to also denote the other geometric form as well as mixtures thereof.

In the context of the present invention, the term "aryl" intends to denote C₅-C₁₈ monocyclic and polycyclic aromatic hydrocarbons with 5 to 18 carbon atoms in the cyclic system. Specifically, this definition comprises, for example, the meanings cyclopentadienyl, phenyl, cycloheptatrienyl, cyclooctatetraenyl, naphthyl and anthracenyl.

In the context of the present invention, the term "heteroaryl" intends to denote C₅-C₁₈ monocyclic and polycyclic aromatic hydrocarbons with 5 to 18 carbon atoms in the cyclic system, wherein one or more methine (-C=) and/or vinylene (-CH=CH-) groups are replaced by trivalent or divalent heteroatoms, in particular nitrogen, oxygen and/or sulphur, respectively, in such a way as to maintain the continuous π-electron system characteristic of aromatic systems. Specifically, this definition comprises, for example, the meanings 2-furyl, 3-furyl, 2-thienyl, 3-thienyl, 2-pyrrolyl, 3-pyrrolyl, 3-isoxazolyl, 4-isoxazolyl, 5-isoxazolyl, 3-isothiazolyl, 4-isothiazolyl, 5-isothiazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl, 1,2,4-oxadiazol-3-yl, 1,2,4-oxadiazol-5-yl, 1,2,4-thiadiazol-3-yl, 1,2,4-thiadiazol-5-yl, 1,2,4-triazol-3-yl, 1,3,4-oxadiazol-2-yl, 1,3,4-thiadiazol-2-yl and 1,3,4-triazol-2-yl; 1-pyrrolyl, 1-pyrazolyl, 1,2,4-triazol-1-yl, 1-imidazolyl, 1,2,3-triazol-1-yl, 1,3,4-triazol-1-yl; 3-pyridazinyl, 4-pyridazinyl, 2-pyrimidinyl, 4-pyrimidinyl, 5-pyrimidinyl, 2-pyrazinyl, 1,3,5-triazin-2-yl and 1,2,4-triazin-3-yl.

In the context of the present invention, the term "C₁-C₁₂-alkyl" is intended to denote straight or branched alkyl groups having one to twelve carbon atoms. The group comprises, for example, methyl, ethyl, n-propyl, isopropyl, n-, iso-, sec-and t-butyl, n-pentyl and its isomers, n-hexyl and its isomers, 1,3-dimethylbutyl, 3,3-dimethylbutyl, n-heptyl and its isomers, n-octyl and its isomers, n-nonyl and its isomers, n-decyl and its isomers, n-undecyl and its isomers and n-dodecyl and its isomers. Often, the C₁ to C₄ alkyl groups are the most preferred groups of the C₁-C₁₂ alkyl group. The term "C₁-C₄-alkyl" is intended to denote straight or branched alkyl groups having one to four carbon atoms. This group comprises methyl, ethyl, n-propyl, isopropyl, n-, iso-, sec- and t-butyl.

In the context of the present invention, the term "C₃-C₈ cycloalkyl" intends to denote mono-, bi- or tricyclic hydrocarbon groups comprising 3 to 10 carbon atoms, especially 3 to 6 carbon atoms. Examples of monocyclic groups include cyclopropyl, cyclobutyl, cyclopentyl, cyclohexyl, cycloheptyl or cyclooctyl. Examples of bicyclic groups include bicyclo[2.2.1]heptyl, bicyclo[3.1.1]heptyl, bicyclo[2.2.2]octyl and bicyclo[3.2.1]octyl. Examples of tricyclic groups are adamantyl and homoadamantyl.

Each of the groups selected from C₁₋₁₂ alkyl, C₃₋₈ cycloalkyl, aryl and heteroaryl can be optionally substituted by one or more substituents selected from the group consisting of R', -X", -OR', -SR', -NR'₂, -SiR'₃, -COOR', -(CO)R', -CN and -CONR'₂, wherein R' is selected independently, from the group consisting of hydrogen or C₁-C₁₂-alkyl group and X" is selected from the group consisting of F, Cl, Br, or I.

In one aspect, the invention concerns a process for the manufacture of a compound of formula (I) 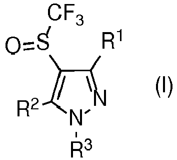
wherein R¹ is a group selected from the group consisting of -CN and COOR⁴
wherein R² is a group selected from the group consisting of -NO₂ and NH₂
wherein R³ is an optionally substituted aryl or an optionally substituted heteroaryl group
wherein R⁴ is selected from the group consisting of H, C₁₋₁₂ alkyl, C₃₋₈-cycloalkyl, aryl or heteroaryl, all of which can be optionally substituted
which comprises at least one of the steps (a) to (d), wherein
in (a), a compound of formula (II) is reacted with CF₃SO₂X, wherein X is selected from the group consisting of F, Cl and Br to obtain a compound of formula (III), which is subsequently reduced with a reducing agent in order to obtain the compound of formula (I) 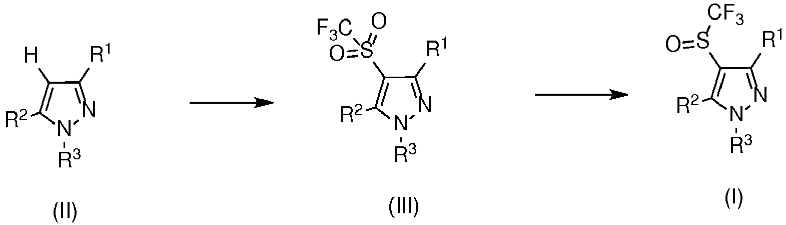
in (b) a compound of formula (IV) CF₃CO₂M, wherein M is selected from Li, K and Na, is reacted with SO₂ to obtain a compound of formula (V) CF₃SO₂M, wherein M is defined as above; wherein (V) is reacted with a compound of formula R⁵X, wherein R⁵ is selected from the group consisting of C₁₋₁₂ alkyl, C₃₋₈-cycloalkyl, aryl and heteroaryl, all of which are optionally substituted, and X is selected from the group consisting of F, Cl, Br and I, to obtain a compound of formula (VI), which is reacted with a compound of formula (II) to obtain a compound of formula (I),
in (c), a compound of formula (IV) CF₃CO₂M, wherein M is selected from Li, K and Na, is reacted with SO₂ to obtain a compound of formula (V) CF₃SO₂M, wherein M is defined as above; wherein subsequently, (V) is reacted with Me₃SiX, wherein X is selected from the group consisting of F, Cl, Br and I, to obtain a compound of formula (VII) CF₃SO₂SiMe₃; wherein (VII) subsequently is reacted with a compound of formula (II) in the presence of SOX'₂, wherein X' is selected from Cl and Br, to obtain the compound of formula (I),
in step (d), the compound of formula (II) is reacted with a compound SX₂, followed by a reaction with a chlorodifluoroacetic ester, in the presence of a fluoride source, and a suitable catalyst, followed by an oxidation step to obtain the compound of formula (I)

In the present invention, the denotion "M" includes "M⁺", such as K⁺, Na⁺ and Li⁺. It is understood that the counterpart of "M⁺" in the compound of formula (IV) is an anion of formula CF₃CO₂⁻, and that the counterpart of "M⁺" in the compound of formula (V) is an anion of formula CF₃SO₂⁻.

In the process for the manufacture of a compound of formula (I), R³ is optionally substituted phenyl, wherein preferably, R³ is a phenyl residue substituted with one or more of the groups selected from CF₃, X, wherein X"' is selected from F, Cl and B, CN and NH₂, and wherein most preferably, R³ is 2,6-dichloro-4-(trifluoromethyl)phenyl.

In one aspect of the process for the manufacture of a compound of formula (I), R¹ preferably is -CN.

In another aspect in the process for the manufacture of a compound of formula (I), R² preferably is -NH₂.

In in the process for the manufacture of a compound of formula (I), in step (b) or (c), preferably M is K.

In the process for the manufacture of a compound of formula (I), in step (b), R⁵ in R⁵X preferably is a C₁₋₄ alkyl group, wherein methyl and ethyl are most preferred, and X in R⁵X preferably is Cl or Br, wherein Cl is most preferred.

In one aspect of the process for the manufacture of a compound of formula (I), in step (c), X in Me₃SiX preferably is Cl, and X' in SOX'₂ preferably is Cl. In the process for the manufacture of a compound of formula (I), in step (c), the reducing agent is selected from the group consisting of NaBH₄, LiAlH₄, NaH, diisobutylaluminium hydride, silyl hydrides, phosphorous hydrides such as phosphine, Raney nickel and H₂ and Raney nickel in formic acid, wherein NaBH₄ and LiAlH₄ are preferred.

In step (d), X in SX₂ is selected from F, Cl, Br and I, wherein SX₂ preferably is SCl₂. The intermediate compound (X) is then reacted with a chlorodifluoroacetic ester, such as chlorodifluoroacetic methylester or chlorodifluoroacetic ethylester, in the presence of a fluoride source, such as KF or AgF, and a suitable catalyst, such as CuI. DMF is a preferred solvent for such a conversion. The intermediate compound of formula (XI) is then submitted to an oxidation reaction with a suitable oxidant, for example 3-chloroperbenzoic acid (mCPBA) or H₂O₂, to obtain the compound of formula (I).

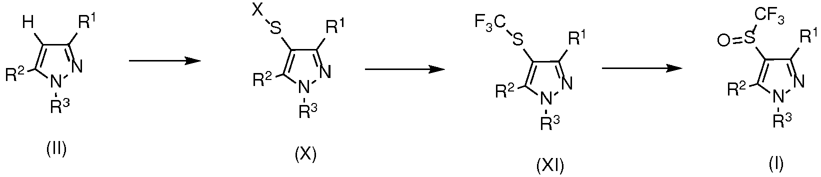

According to the present invention, an auxiliary base can be present in any of steps (a), (b) or (c), for example to act as an acid scavenger to scavenge evolving hydrogen halide or other acidic by-products. Such auxiliary bases can be organic or inorganic, such as for example Et₃N or pyridine, or for example KOH, NaOH or LiOH.

According to the present invention, the manufacture for the compound of formula (I) can further comprise a step wherein the group R¹ is COOR⁴, wherein R⁴ preferably is a H or a C₁₋₄ alkyl group, in the compound of formula (I), (II) or (III), and wherein R¹ is converted into a group R¹ which is -CN. When R⁴ is H, the transformation of group -COOH can be achieved, for example, by reaction of compound (I), (II) or (III) with n-propylphosphonic anhydride and at least one suitable nitrogen source such as Et₃N and 2- methylpropan-2-amine followed by a reaction with POCl₃, phosphorus pentachloride or POCl₃ and ammonia, ammonia or ammonia salts and optionally an auxiliary such as pyridine and/or di-tert-butyl dicarbonate followed by a dehydrating agent such as trifluoroacetic anhydride, or other conversion methods suitable to achieve the conversion of - COOH to -CN, such as reaction with CuCN. When R⁴ is C₁₋₁₂ alkyl, C₃₋₈-cycloalkyl, aryl or heteroaryl, wherein C₁ to C₄ alkyl is preferred, -COOR⁴ is often converted into -COOH by saponification prior to a reaction as described above to obtain the compound according to formula (I), (II) or (III) with R¹ = CN. In another aspect, -COOR⁴ with R⁴ being selected from C₁₋₁₂ alkyl, C₃₋₈-cycloalkyl, aryl or heteroaryl, wherein C₁ to C₄ alkyl is preferred, is reacted with an ammonia source, such as NH₃, NH₄OH or NH₄HCO₃, followed by a dehydration agent such as POCl₅, trifluoroacetic anhydride or PCl₅. In another aspect, it can be advantageous to convert the group -COOH into a group -C(O)X prior to conversion into -CN.

In another aspect of the process of the present invention for the manufacture of a compound of formula (I), the process comprises a step wherein the group R² is NO₂ in the compound of formula (I), (II) or (III), and wherein R² is converted into a group R² which is -NH₂. The transformation of the group - NO₂ into -NH₂ can be effected by a suitable reducing agent, for example NaBH₄, silyl hydrides, phosphorous hydrides such as phosphine, LiAlH₄, H₂ / Pd or H₂ / Raney Nickel.

Another object of the present invention is a process for the manufacture of a compound of formula (VIII) wherein R¹ is CN and R² is NH₂, wherein a compound of formula (IX), wherein R³ is defined as above, is reacted with 3-amino-3-oxopropanoyl cyanide

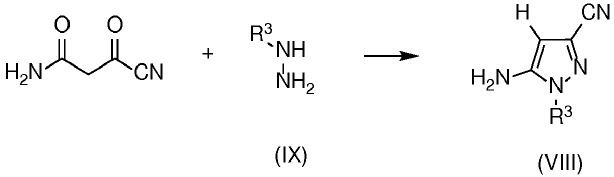

The compound of formula (IX) can also be used in the form of a salt of compound (IX), such as its hydrohalide salt, or an aqueous solution of compound (IX). The process for the manufacture of a compound of formula (VIII) can also comprise a further step of reacting 3-amino-3-oxopropanoic acid with CuCN in order to obtain 3-amino-3-oxopropanoyl cyanide. In one aspect, the 3-amino-3-oxopropanoic acid is converted into its acid halide, preferably into its acid chloride, prior to the reaction with CuCN. In this aspect, it is preferred that the amino function of the 3-amino-3-oxopropanoyl cyanide is protected by a N-protecting group, such as N-acetyl/N-acyl (e.g. Boc group) or isocyanide, prior to conversion of the -COOH group into the -CN group. The processes for the manufacture of the compound according to formula (I) as described above can comprise the process for the manufacture of a compound of formula (VIII) and/or the reaction of 3-amino-3-oxopropanoic acid (or its N-protected derivative) with CuCN in order to obtain 3-amino-3-oxopropanoyl cyanide. In the process for the manufacture of (VIII), R³ preferably is optionally substituted phenyl, more preferably, R³ is a phenyl residue substituted with one or more of the groups selected from CF₃, X, wherein X"' is selected from F, Cl and B, CN and NH₂, and most preferably, R³ is 2,6-dichloro-4-(trifluoromethyl)phenyl.

The invention further concerns a process for the manufacturing of an agriculturally, insecticidal or pharmaceutically active compound or a composition comprising an agriculturally, insecticidal or pharmaceutically active compound, which comprises at least one of the processes for the manufacture of a compound of formula (I) and/or (VIII), wherein the agriculturally, insecticidal or pharmaceutically active compound preferably is 5-amino-1-(2,6-dichloro-4-trifluoromethylphenyl)-3-cyano-4-trifluoromethylsulfinylpyrazole or a precursor thereof.

The invention also concerns the compound 3-amino-3-oxopropanoyl cyanide, which is a useful precursor for agriculturally, insecticidal or pharmaceutically active compounds, for example a precursor for a compound of formula (VIII), or any of its derivatives wherein its amino function is protected, for example by N-acetyl/N-acyl (e.g. Boc group) or isocyanide.

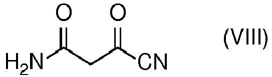

The processes according to the present invention give access to agriculturally, insecticidal or pharmaceutically active compounds or precursors thereof, often providing improved yields, easier process handling and cheaper starting materials than other processes. Waste production and energy consumption in these processes can also be reduced.

Should the disclosure of any patents, patent applications, and publications which are incorporated herein by reference conflict with the description of the present application to the extent that it may render a term unclear, the present description shall take precedence.

The following examples are intended to further explain the invention without limiting it.

The starting materials for the processes and steps according to the present invention can be obtained from commercial sources or can be manufactured according to procedures known from the public domain. Potassium triflinate can be obtained, for example, by the method described in WO2015036504. Trimethylsilyl trfluoromethanesulfonate can be obtained, for example, by the method described in CN102911196. The compound of formula (VIII), in particular 5-amino-1-(2,6-dichloro-4-(trifluoromethyl)phenyl)-1H-pyrazole-3-carbonitrile, can be obtained according to the process of the present invention, or, for example, according to the procedures described in WO2008005489. 3-amino-3-oxopropanoic acid (malonamic acid) can be prepared by LiOH hydrolysis of the commercially available malonamic acid ethyl ester.

### Example 1 tert-butyl (3-cyano-3-oxopropanoyl)carbamate .

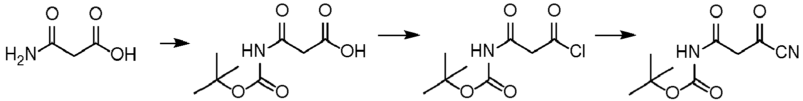

0.024 mol 3-amino-3-oxopropanoic acid are dissolved in 70 ml acetonitrile. 3.7 g di- tert-butyl-dicarbonate and 900 mg 4-dimethylaminopyridine are added thereto. After stirring for 24 hours at ambient temperature the mixture is divided between 1 N HCl and ethyl acetate. The aqueous phase is extracted with ethyl acetate and the combined organic phases are washed with saturated sodium hydrogen carbonate solution and saturated sodium chloride solution. After drying with magnesium sulphate the solvents are eliminated in vacuo to yield the crude 3-((tert-butoxycarbonyl)amino)-3-oxopropanoic acid.

To a solution of crude ((3,3-di-(tert-butoxycarbonyl))amino)-3-oxopropanoic acid (0.150 moles) in dichloromethane (50 mL) is slowly added oxalyl chloride (19.6 mL, 0.225 moles) at 0 °C. The reaction mixture is stirred for 5 h and then the solvent is removed under reduced pressure without heating to give the crude tert-butyl (3-chloro-3-oxopropanoyl)carbamate.

To a solution of the crude 3((3,3-di-(tert-butoxycarbonyl))amino)-3-oxopropanoyl chloride (15.6 mmole) in acetonitrile (30 mL) is added anhydrous copper(I)cyanide (2.79 g, 31.1 mmole), and the suspension is stirred at 90 °C for 1.5 h. The solution is cooled down to room temperature, the mixture is filtered over Celite(R) and the solvent is removed under reduced pressure to yield the crude tert-butyl (3-cyano-3-oxopropanoyl)carbamate

### Example 2 tert-butyl (3-cyano-1-(2,6-dichloro-4-(trifluoromethyl)phenyl)-1H-pyrazol-5-yl)carbamate

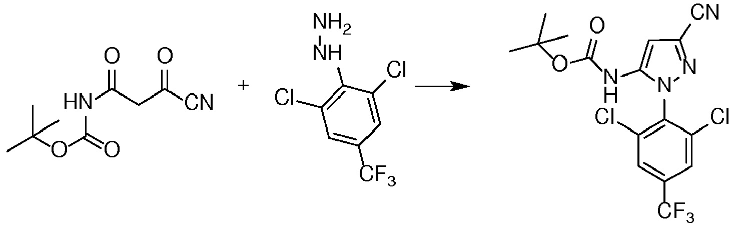

tert-butyl (3-cyano-3-oxopropanoyl)carbamate (0.15 moles) is solved in 150 mL dichloromethane. (2,6-dichloro-4-(trifluoromethyl)phenyl)hydrazine (0.21 mole) in 50 mL dichloromethane are added at 10°C, followed by 0.15 mole trimethylamine, while keeping the temperature at 10°C. The mixture is stirred at 20°C for 5 hours, washed with water and brine, dried over Na₂SO₄ and the solvent are evaporated to give the crude product.

### Example 3 5-amino-1-(2,6-dichloro-4-(trifluoromethyl)phenyl)-4-((trifluoromethyl)sulfonyl)-1H-pyrazole-3-carbonitrile

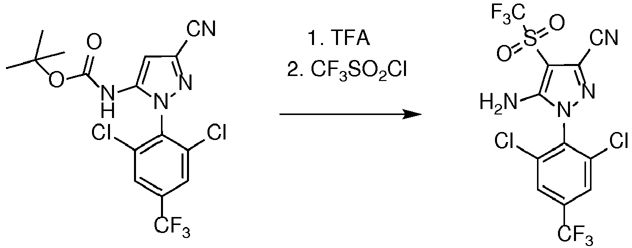

tert-butyl (3-cyano-1-(2,6-dichloro-4-(trifluoromethyl)phenyl)-1H-pyrazol-5-yl)carbamate (0.351 mole) is solved in 100 mL DCM, mixed with 10 mL trifluoroacetic acid at room temperature, stirred for one hour at room temperature, and all volatiles were evaporated. The residue is taken up into EtOAc, washed with aw. NaHCO₃, dried over Na₂SO₄, and the volatiles are evaporated. The crude deprotected intermediate is solved in 300 mL dry dichloromethane, and under nitrogen atmosphere, trifluoromethyl sulfonyl chloride 80.3 g (0.526 moles) are added at 15-20°C. The reaction mass is stirred for 12 hours at 20-30°C and finally neutralized with caustic solution. The organic phase is separated, the aq. phase extracted twice with dichloromethane, the organic phases combined, dried over MgSO4, and the solvent evaporated to obtain the crude product.

### Example 4 5-amino-1-(2,6-dichloro-4-(trifluoromethyl)phenyl)-4-((trifluoromethyl)sulfinyl)-1H-pyrazole-3-carbonitrile

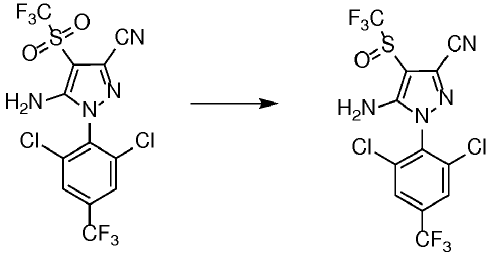

To a solution of 5-amino-1-(2,6-dichloro-4-(trifluoromethyl)phenyl)-4-((trifluoromethyl)sulfonyl)-1H-pyrazole-3-carbonitrile (1.1 mmole) in tetrahydrofuran is added sodium hydride (60%, 0.06 g, 1.4 mmole). The mixture is heated at reflux for 10 hours. After extractive workup and removal of the volatiles under vacuum, the crude product is obtained.

## Claims

1. Process for the manufacture of a compound of formula (I) 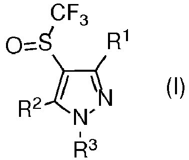
wherein R¹ is a group selected from the group consisting of -CN and COOR⁴
wherein R² is a group selected from the group consisting of -NO₂ and NH₂
wherein R³ is an optionally substituted aryl or an optionally substituted heteroaryl group
wherein R⁴ is selected from the group consisting of H, C₁₋₁₂ alkyl, C₃₋₈-cycloalkyl, aryl or heteroaryl, all of which can be optionally substituted
which comprises at least one of the steps (a) to (d), wherein
in (a), a compound of formula (II) is reacted with CF₃SO2X, wherein X is selected from the group consisting of F, Cl, Br and I, to obtain a compound of formula (III), which is subsequently reduced with a reducing agent in order to obtain the compound of formula (I) 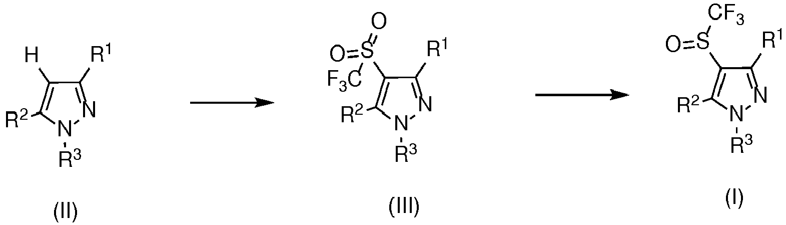
in (b), a compound of formula (IV) CF₃CO₂M, wherein M is selected from Li, K and Na, is reacted with SO₂ to obtain a compound of formula (V) CF₃SO₂M, wherein M is defined as above; wherein (V) is reacted with a compound of formula R⁵X, wherein R⁵ is selected from the group consisting of C₁₋₁₂ alkyl, C₃₋₈-cycloalkyl, aryl and heteroaryl, all of which are optionally substituted, and X is selected from the group consisting of F, Cl, Br and I, to obtain a compound of formula (VI), which is reacted with a compound of formula (II) to obtain a compound of formula (I),
in (c), a compound of formula (IV) CF₃CO₂M, wherein M is selected from Li, K and Na, is reacted with SO₂ to obtain a compound of formula (V) CF₃SO₂M, wherein M is defined as above; wherein subsequently, (V) is reacted with Me₃SiX, wherein X is selected from the group consisting of F, Cl, Br and I, to obtain a compound of formula (VII) CF₃SO₂SiMe₃; wherein (VII) subsequently is reacted with a compound of formula (II) in the presence of SOX'₂, wherein X' is selected from Cl and Br, to obtain the compound of formula (I)
in (d), the compound of formula (II) is reacted with a compound SX₂ wherein X is selected from F, Cl, Br and I, to obtain an intermediate compound of formula (X), followed by a reaction with a chlorodifluoroacetic ester, in the presence of a fluoride source, and a suitable catalyst to obtain intermediate compound of formula (XI), followed by an oxidation step to obtain the compound of formula (I)

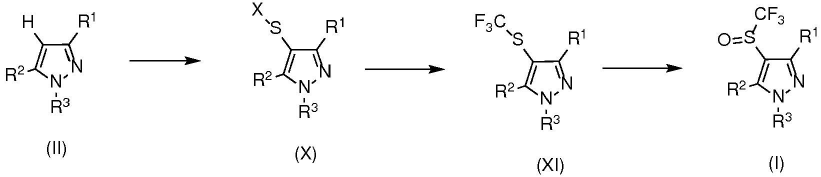

2. Process according to claim 1, wherein R³ is optionally substituted phenyl.

3. Process according to claim 2, wherein R³ is a phenyl residue substituted with one or more of the groups selected from CF₃, X, wherein X"' is selected from F, Cl and B, CN and NH₂.

4. Process according to claim 3, wherein R³ is 2,6-dichloro-4-(trifluoromethyl)phenyl.

5. Process according anyone of claims 1 to 4, wherein R¹ is -CN.

6. Process according anyone of claims 1 to 5, wherein R² is -NH₂.

7. Process according anyone of claims 1 to 6, wherein in step (b), R⁵ in R⁵X is a C₁₋₄ alkyl group, and X in R⁵X preferably is Cl or Br.

8. Process according anyone of claims 1 to 7, wherein in step (c), the reducing agent is selected from the group consisting of NaBH₄, LiAlH₄, NaH, diisobutylaluminium hydride, Raney nickel and H₂ and Raney nickel in formic acid.

9. Process according to anyone of claims 1 to 8, which comprises a step wherein the group R¹ = COOR⁴ in the compound of formula (I), (II) or (III) is converted into a group R¹ which is -CN.

10. Process according to anyone of claims 1 to 9, which comprises a step wherein the group R² = NO₂ in the compound of formula (I), (II) or (III) is converted into a group R² which is -NH₂.

11. Process for the manufacture of a compound of formula (VIII) wherein R¹ is CN and R² is NH₂, wherein a compound of formula (IX), wherein R³ is defined as in anyone of claims 1 to 7, is reacted with 3-amino-3-oxopropanoyl cyanide or an N-protected derivative thereof.

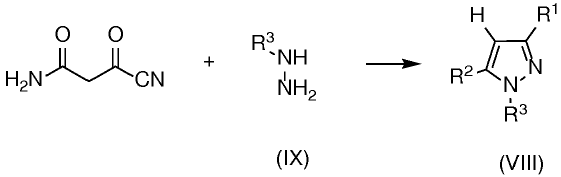

12. Process according to claim 11, which further comprises reacting 3-amino-3-oxopropanoic acid with CuCN in order to obtain 3-amino-3-oxopropanoyl cyanide.

13. The compound 3-amino-3-oxopropanoyl cyanide or an N-protected derivative thereof.

14. Process according to anyone of claims 1 to 11, which further comprises a process according to claim 12 or 13.

15. Process for the manufacturing of an agriculturally, insecticidal or pharmaceutically active compound or a composition comprising an agriculturally or pharmaceutically active compound, which comprises at least one of the processes according to anyone of claims 1 to 14.
